# EUROPÄISCHE PATENTSCHRIFT

(11) **EP 0 299 470 B1**
(45) Veröffentlichungstag und Bekanntmachung des Hinweises auf die Patenterteilung: **04.12.1996**
(21) Anmeldenummer: 88111302.1
(22) Anmeldetag: 14.07.1988
(51) Int. Cl.: C07D 471/04, A61K 31/435

(54) **Imidazo[1,2-a]pyridine**
Imidazo[1,2-a]pyridines
Imidazo[1,2-a]pyridines

(30) Priorität: 16.07.1987 CH 2709/87; 04.02.1988 CH 390/88
(43) Veröffentlichungstag der Anmeldung: 18.01.1989
(73) Patentinhaber: Byk Gulden Lomberg Chemische Fabrik GmbH, D-78403 Konstanz (DE)
(72) Erfinder: Senn-Bilfinger, Jörg, Dr., D-7750 Konstanz (DE); Grundler, Gerhard, Dr., D-7750 Konstanz (DE); Schaefer, Hartmann, Dr., D-7750 Konstanz (DE); Klemm, Kurt, Prof.Dr., D-7753 Allensbach (DE); Rainer, Gerog, Dr., D-7750 Konstanz (DE); Schudt, Christian, Dr., D-7750 Konstanz (DE); Simon, Wolfgang-Alexander, Dr., D-7750 Konstanz (DE); Riedel, Richard, Dr., D-7750 Konstanz (DE); Postius, Stefan, Dr., D-7750 Konstanz (DE)

(56) Entgegenhaltungen:
- EP-A- 0 033 094
- EP-A- 0 204 285

## Beschreibung

### Anwendungsgeblet der Erfindung

Die Erfindung betrifft neue Imidazo[1,2a]pyridine; Verfahren zu ihrer Herstellung, ihre Anwendung und sie enthaltende Arzneimittel. Die erfindungsgemäßen Verbindungen werden in der pharmazeutischen Industrie als Zwischenprodukte und zur Herstellung von Medikamenten verwendet.

### Bekannter technischer Hintergrund

Aus den veröffentlichten Europäischen Patentanmeldungen 0 033 094, 0 068 378, 0 165 545 und 0 204 285 sind Imidazopyridine bzw. Imidazoisochinoline bekannt, die aufgrund ihrer antisekretorischen und cytoprotektiven Wirkung bei der Behandlung von Ulcuserkrankungen eingesetzt werden sollen.

### Beschreibung der Erfindung

Es wurde nun gefunden, daß die unten näher beschriebenen neuen Imidazo[1,2-a]pyridin verbindungen interessante pharmakologische Eigenschaften aufweisen, durch die sie sich von den obenerwähnten bekannten Verbindungen in überraschender und besonders vorteilhafter Weise unterscheiden.

Gegenstand der Erfindung sind neue Imidazo[1,2-a]pyridin verbindungen der Formel I 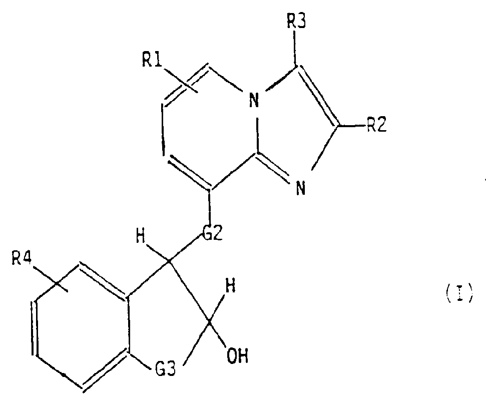 worin
R1 Wasserstoff (H) oder Halogen,
R2 1-4C-Alkyl,
R3 Wasserstoff (H), 1-4C-Alkyl, Formyl, Hydroxy-1-4C-alkyl, Cyano-1-4C-alkyl , Nitroso oder Amino,
R4 Wasserstoff (H) oder Halogen,
G2 0 (Sauerstoff) oder NH und
G3 -CH₂- oder -CH₂CH₂- bedeutet,
   wobei G2 und OH trans-ständig zueinander stehen, und ihre Salze.
   Halogen im Sinne der vorliegenden Erfindung ist Brom und insbesondere Chlor und Fluor.
   1-4C-Alkyl steht für geradkettige oder verzweigte Alkylreste mit 1 bis 4 Kohlenstoffatomen. Beispielsweise seien genannt der Butyl-, iso-Butyl-, sec-Butyl- tert.-Butyl-, Propyl-, Isopropyl-, Ethyl- und insbesondere der Methylrest.
   Hydroxy-1-4C-alkyl steht für die vorstehend genannten 1-4C-Alkylreste, an die ein Hydroxylrest gebunden ist. Bevorzugt ist der Hydroxymethylrest.
   Cyano-1-4C-alkyl steht für die vorstehend genannten 1-4C-Alkylreste, an die ein Cyanrest gebunden ist. Bevorzugt ist der Cyanomethylrest.

Als Salze kommen für Verbindungen der Formel einerseits bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosylat, 2-Hydroxy-3-naphthoat, 3-Hydroxy-2-naphthoat oder Mesylat.

Weiterhin kommen als Salze alle Alkoholate in Betracht, die durch Umsetzung der Verbindungen der Formel mit geeigneten Deprotonierungsmitteln (z.B. starken Basen) erhalten werden können. Als geeignete Deprotonierunqsmittel seien beispielsweise metallorganische Verbindungen, wie Butyllithium, oder Alkalihydride, wie Natriumhydrid, genannt.

Die erfindungsgemäßen Verbindungen haben zumindest zwei asymmetrische Kohlenstoffatome. Die Erfindung betrifft die Konfigurationskombinationen, bei denen G2 und OH trans-ständig zueinander stehen. Bevorzugter Gegenstand der Erfindung sind die optisch reinen Verbindungen der Formel I.

Erwähnenswerte erfindungsgemäße Verbindungen sind solche der Formel I, worin
R1 Wasserstoff, Chlor oder Fluor,
R2 Methyl oder Ethyl,
R3 Wasserstoff, Methyl, Formyl, Hydroxymethyl, Cyanomethyl oder Amino,
R4 Wasserstoff, Chlor oder Fluor,
G2 0 (Sauerstoff) oder NH und
G3 -CH₂- oder -CH₂CH₂- bedeutet,

### und ihre Salze.

Bevorzugte erfindungsgemäße Verbindungen sind solche der Formel 1 , worin
R1 Wasserstoff,
R2 Methyl,
R3 Wasserstoff, Methyl, Hydroxymethyl, Cyanomethyl oder Amino,
R4 Wasserstoff oder Fluor,
G2 0 (Sauerstoff) oder NH und
G3 _CH₂- oder -CH₂CH₂- bedeutet,

### und ihre Salze.

Ausgewählte erfindungsgemäße Verbindungen können durch die folgenden Formeln la und Ib charakterisiert werden, 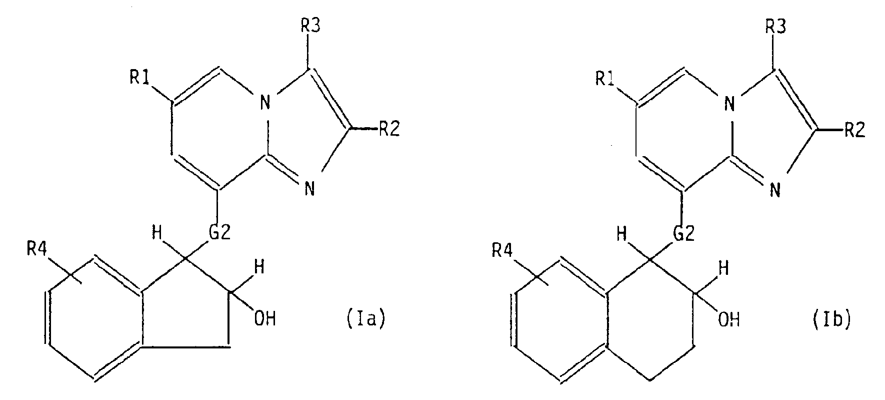 worin die Substituenten und Symbole R1, R2, R3, R4 und G2 die oben angegebenen Bedeutungen haben. Unter Berücksichtigung der absoluten Konfiguration an den Positionen 1' und 2' im Dihydroindenyl- bzw Tetrahydronaphthylrest ergeben sich für die ausgewählten Verbindungen die folgenden besonders bevorzugten optisch reinen Konfigurationsisomeren; 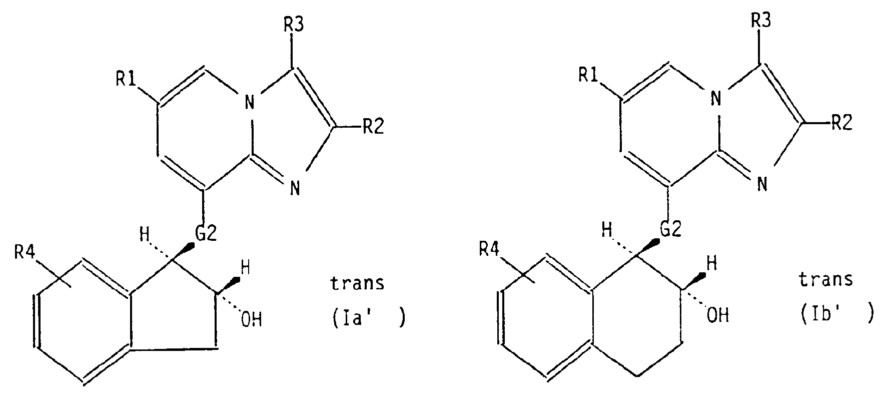 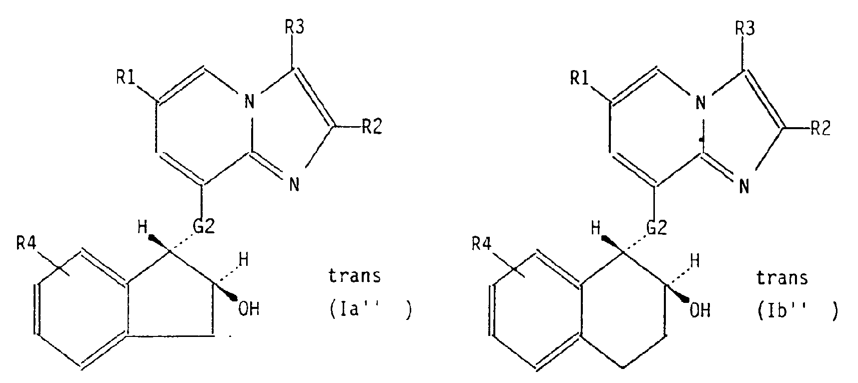 worin R1, R2, R3, R4 und G2 die oben angegebenen Bedeutungen haben

Als besonders hervorzuhebende erfindungsgemäße Verbindungen seien beispielsweise genannt:
8-(2-Hydroxy-1,2, 3,4-tetrahydro-1-naphthyloxy)-2-methyl-imidazo[1,2-a]pyridin
8-(2-Hydroxy-1,2, 3,4-tetrahydro-1-naphthyloxy)-2,3-dimethyl-imidazo[1,2-a]pyridin
8-(2-Hydroxy-1,2, 3,4-tetrahydro-1-naphthyloxy)-3-hydroxymethyl-2-methyl-imidazo[1,2-a]pyridin
3-Cyanomethyl-8-(2-hydroxy-1,2,3,4-tetrahydro-1-naphthyloxy)-2-methyl-imidazo[1,2-a]pyridin
E-(2-Hydroxy-1,2,3,4-tetrahydro-1-naphthylamino)-2-methyl-imidazo[1,2-a]pyridin 8-(2-Hydroxy-1,2,3,4-tetrahy- dro-1-naphthylamino)-2,3-dimethyl-imidazo[1,2-a]pyridin
3-Hydroxymethyl-8-(2-hydroxy-1,2,3,4-tetrahydro-1-naphthylamino)-2-methyl-imidazo[1,2-a]pyridin
3-Cyanomethyl-8-(2-hydroxy-1,2,3;4-tetrahydro-1-naphthylamino)-2-methyl-imidazo[1,2-a]pyridin
3-Amino-8-(2-hydroxy-1,2,3,4-tetrahydro-1-naphthyloxy)-2-methyl-imidazo[1,2-a]pyridin
3-Amino-8-(2-hydroxy-2,3-dihydro-1-indenyloxy)-2-methyl-imidazo[1,2-a]pyridin
8-(2-Hydroxy-2,3-dihydro-1-indenyloxy)-2-methyl-imidazo[1,2-a]pyridin
8-(2-Hydroxy-2,3-dihydro-1-indenyloxy)-2,3-dimethyl-imidazo[1,2-a]pyridin
3-Hydroxymethyl-8-(2-hydroxy-2,3-dihydro-1-indenyloxy)-2-methyl-imidazo[1,2-a]pyridin
2-Ethyl-3-hydroxymethyl-8-(2-hydroxy-2,3-dihydro-1-indenyloxy)-imidazo[1,2-a]pyridin
3-Cyanomethyl-8-(2-hydroxy-2,3-dihydro-1-indenyloxy)-2-methyl-imidazo[1,2-a]pyridin
3-Amino-8-(2-hydroxy-2,3-dihydro-1-indenylamino)-2-methyl-imidazo[1,2-a]pyridin
8-(2-Hydroxy-2,3-dihydro-1-indenylamino)-2-methyl-imidazo[1,2-a]pyridin
8-(2-Hydroxy-2,3-dihydro-1-indenylamino)-2,3-dimethyl-imidazo[1,2-a]pyridin
3-Hydroxymethyl-8-(2-hydroxy-2,3-dihydro-1-indenylamino)-2-methyl-imidazo[1,2-a]pyridin
3-Cyanomethyl-8-(2-hydroxy-2,3-dihydro-1-indenylamino)-2-methyl-imidazo[1,2-a]pyridin
2-Hydroxymethyl-8-(2-hydroxy-2,3-dihydro-1-indenylamino)-3-methyl-imidazo[1,2-a]pyridin
3-Amino-8-(6-fluor-2-hydroxy-1,2,3,4-tetrahydro-1-naphthyloxy),-2-methyl-imidazo[1,2-a]pyridin
8-(6-Fluor-2-hydroxy-1,2,3,4-tetrahydro-1-naphthyloxy)-2-methyl-imidazo[1,2-a]pyridin
8-(6-Fluor-2-hydroxy-1,2,3,4-tetrahydro-1-naphthyloxy)-2,3-dimethyl-imidazo[1,2-a]pyridin
8-(6-Fluor-2-hydroxy-1,2,3,4-tetrahydro-1-naphthyloxy)-3-hydroxymethyl-2-methyl-imidazo[1,2-a]pyridin
3-Cyanomethyl-8-(6-fluor-2-hydroxy-1,2,3,4-tetrahydro-1-naphthyloxy)-2-methylimidazo[1,2-a]pyridin
8-(6-Fluor-2-hydroxy-1,2,3,4-tetrahydro-1-naphthylamino)-2-methyl-imidazo[1,2-a]pyridin
8-(6-Fluor-2-hydroxy-1,2,3,4-tetrahydro-1-naphthylamino)-2,3-dimethyl-imidazo[1,2-a]pyridin
8-(6-Fluor-2-hydroxy-1,2,3,4-tetrahydro-1-naphthylamino)-3-hydroxymethyl-2-methyl-imidazo[1,2-a]pyridin
3-Cyanomethyl-8-(6-fluor-2-hydroxy-1,2,3,4-tetrahydro-1-naphthylamino)-2-methyl-imidazo[1,2-a]pyridin
8-(5-Fluor-2-hydroxy-2,3-dihydro-1-indenyloxy)-2-methyl-imidazo[1,2-a]pyridin 3-Amino-8-(5-fluor-2-hydroxy-2,3-dihydro-1 -indenyloxy)-2-methyl-imidazo[1,2-a]pyridin
8-(5-Fluor-2-hydroxy-2,3-dihydro-1-indenyloxy)-2,3-dimethyl-imidazo[1,2-a]pyridin
8-(5-Fluor-2-hydroxy-2,3-dihydro-1-indenyloxy)-3-hydroxymethyl-2-methyl-imidazo[1,2-a]pyridin
2-Ethyl-8-(5-fluor-2-hydroxy-2,3-dihydro-1-indenyloxy)-3-hydroxymethyl-imidazo[1,2-a]pyridin
3-Cyanomethyl-8-(5-fluor-2-hydroxy-2,3-dihydro-1-indenyloxy)-2-methyl-imidazo[1,2-a]pyridin
8-(5-Fluor-2-hydroxy-2,3-dihydro-1-indenylamino)-2-methyl-imiGazo[1,2-a]pyridin 8-(5-Fluor-2-hydroxy-2,3-dihy- dro-1-indenylamino)-2,3-dimethyl-imidazo[1,2-a]pyridin
8-(5-Fluor-2-hydroxy-2,3-dihydro-1-indenylamino)-3-hydroxymethyl-2-methyl-imidazo[1,2-a]pyridin
3-Cyanomethyl-8-(5-fluor-2-hydroxy-2,3-dihydro-1-indenylamino)-2-methyl-imidazo[1,2-a]pyridin
3-Amino-8-(5-chlor-2-hydroxy-2,3-dihydro-1-indenyloxy)-2-methyl-imidazo[1,2-a]pyridin
3-Amino-8-(4-fluor-2-hydroxy-2,3-dihydro-1-indenyloxy)-2-methyl-imidazo[1,2-a]pyridin
3-Amino-8-(6-fluor-2-hydroxy-2,3-dihydro-1-indenyloxy)-2-methyl-imidazo[1,2-a]pyridin
3-Amino-8-(4-chlor-2-hydroxy-2,3-dihydro-1-indenyloxy)-2-methyl-imidazo[1,2-a]pyridin
3-Amino-8-(6-chlor-2-hydroxy-2,3-dihydro-1-indenyloxy)-2-methyl-imidazo[[1,2-a]pyridin
3-Amino-8-(7-chlor-2-hydroxy-2,3-dihydro-1-indenyloxy)-2-methyl-imidazo[1,2-a]pyridin
8-(5-Chlor-2-hydroxy-2,3-dihydro-1-indenyloxy)-3-hydroxymethyl-2-methyl-imidazo[1,2-a]pyridin
8-(4-Fluor-2-hydroxy-2,3-dihydro-1-indenyloxy)-3-hydroxymethyl-2-methyl-imidazo[1,2-a]pyridin
8-(6-Fluor-2-hydroxy-2, 3-dihydro-1-indenyloxy)-3-hydroxymethyl-2-methyl-imidazo[1,2-a]pyridin
8-(4-Chlor-2-hydroxy-2,3-dihydro-1-indenyloxy)-3-hydroxymethyl-2-methyl-imidazo[1,2-a]pyridin
8-(6-Chlor-2-hydroxy-2,3-dihydro-1-indenyloxy)-3-hydroxymethyl-2-methyl-imidazo[1;2-a]pyridin
8-(7-Chlor-2-hydroxy-2,3-dihydro-1-indenyloxy)-3-hydroxymethyl-2-methyl-imidazo[1,2-a]pyridin

und die Salze dieser Verbindungen.

Bevorzugt sind die optisch reinen Formen der vorstehend aufgeführten Verbindungen, und ihre Salze.

Weiterer Gegenstand der Erfindung ist ein Verfahren zur Herstellung der erfindungsgemäßen Verbindungen und ihrer Salze. Das Verfahren ist dadurch gekennzeichnet, daß man Imidazopyridine der Formel II mit Bicyclen der Formel III umsetzt, 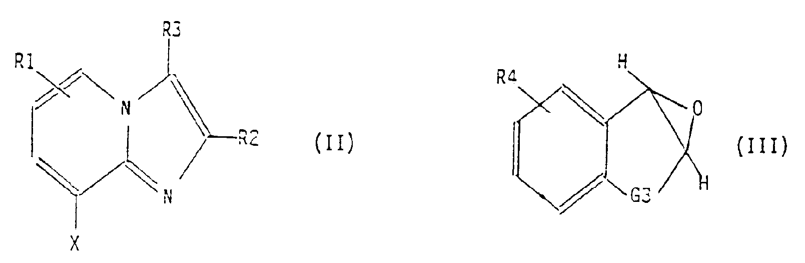 und gewünschtenfalls anschließend erhaltene Verbindungen der Formel worin R3 die Bedeutung Formyl bzw. Nitroso hat, zu den Verbindungen der Formel I, worin R3 die Bedeutung Hydroxymethyl bzw. Amino hat, reduziert, und/oder gewünschtenfalls anschließend erhaltene Salze in die freien Verbindungen oder erhaltene freie Verbindungen in die Salze überführt, wobei die Ausgangsverbindungen als solche oder in Form ihrer Salze eingesetzt werden und wobei R1, R2, R3, R4 und G3 die oben angegebenen Bedeutungen haben und X OH oder NH₂ bedeutet.

Die Reaktionen von II mit III wird in geeigneten, inerten Lösungsmitteln in Gegenwart von Aluminiumoxid oder bevorzugt in polaren, protonenhaltigen Medien in Gegenwart von Basen durchgeführt. Als protonenhaltige Medien kommen Wasser und Alkohole, vorzugsweise Methanol, alleine oder in Zumischung in Frage. Als Basen seien Alkali-oder Erdalkalihydroxide, vorzugsweise Alkalicarbonate und tertiäre organische Amine genannt. Sie können je nach Art im Überschuß oder Unterschuß eingesetzt werden, vorzugsweise in einer Menge von 0,1 - 2 Mol. Die Umsetzung in polaren Medien wird bei Temperaturen von 0° bis 100°C, vorzugsweise bei 20° bis 60°C durchgeführt

Die Herstellung der Epoxide aus geeigneten Vorstufen, z.B. Halogenalkoholen, kann auch in situ erfolgen und im Eintopfverfahren mit deren Umsetzung mit Verbindungen II kombiniert werden, indem wenigstens ein weiteres Mol Base zur Anwendung kommt. - Die Herstellung der erfindungsgemäßen Verbindungen erfolgt bevorzugt durch Umsetzung der Imidazopyridine II (mit X = OH oder NH₂) mit 1,2-Epoxiden, wobei fast ausschließlich das trans-Additionsprodukt entsteht.

Die Reduktion der Verbindungen I, worin R3 die Bedeutung Formyl hat, zu den Verbindungen I, worin R3 die Bedeutung Hydroxymethyl hat, erfolgt auf eine dem Fachmann geläufige Weise, z.B. durch Umsetzung der Formylverbindung mit Natriumborhydrid..

Die Reduktion der Verbindungen I, worin R3 die Bedeutung Nitroso hat, zu den Verbindungen I, worin R3 die Bedeutung Amino hat, erfolgt auf eine dem Fachmann geläufige Weise, z.B. durch Umsetzung der Nitrosoverbindung mit Zinkpulver in saurem Medium.

Welche Reaktionsbedingungen für die Durchführung des Verfahrens im einzelnen erforderlich sind, ist dem Fachmann aufgrund seines Fachwissens geläufig.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einen der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Alkoholate erhält man beispielsweise durch Umsetzung der erfindungsgemäßen Verbindungen mit Alkalimetallen oder Alkalimetallhydriden.

Enantiomer reine Verbindungen 1 erhält man beispielsweise durch Umsetzung enantiomer reiner Bicyclen II mit den Imidazopyridinen 11. Die entiomerenreinen Verbindungen I mit der trans-Konfiguration (wie z.B. la', la", Ib' und Ib") erhält man als trans-Additionsprodukte bevorzugt durch Umsetzung der entsprechenden enantiomerenreinen Epoxide mit den Imidazopyridinen II.

Die Ausgangsverbindungen II sind literaturbekannt oder können in Analogie zu literaturbekannten Methoden, beispielsweise in Analogie zu J.J.Kaminski et al. [J.Med.Chem. 28, 876 (1985)] oder wie in den Europäischen Patentanmeldungen 33 094 oder 68 378 beschrieben hergestellt werden. Die Ausgangsverbindungen III sind ebenfalls literaturbekannt [siehe z B. G.H. Posner et al., J. Amer. Chem Soc. 99, 8214 (1977); M.N. Akhtar et al., J. Chem. Soc. Perk. Trans. I (1979) 2437; D.R. Boyd et al., J. Chem. Soc. Perk. Trans. I (1982) 2767 sowie Bull. Soc. Chim. (France) 11, 3092-3095 (1973)] oder sie können ebenfalls in Analogie zu literaturbekannten Methoden hergestellt werden.

Bevorzugte Ausgestaltungen des Verfahrens sind solche, bei denen die Substituenten und Symbole R1, R2, R3, R4, G2 und G3 die in den Unter- und Nebenansprüchen angegebenen Bedeutungen haben.

Die folgenden Beispiele erläutern die Erfindung näher ohne sie einzuschränken. Die in den Beispielen namentlich aufgeführten Verbindungen der allgemeinen Formel 1 sowie die Salze dieser Verbindungen sind bevorzugter Gegenstand der Erfindung. Schmp. bedeutet Schmelzpunkt, für Stunde(n) wird die Abkürzung h und für Minuten die Abkürzung Min. verwendet. Unter "Ether" wird Diethylether verstanden.

### Beispiele

### Endprodukte

### 1. 8-(2-(trans)-Hydroxy-2,3-dihydro-1-indenylamino)-2-methyl-imidazo[1.2-a]pyridin

Zu einer Suspension von 1 mMol 8-Amino-2-methyl-imidazo[1,2-a]pyridin in trokkenem Diethylether werden 7,5 g Aluminiumoxid (Woelm 200) zugegeben. Nach 30-minütigem Rühren bei Raumtemperatur wird 1 mMol 1,2-Indenoxid zugegeben. Die heterogene Mischung wird 2 bis 48 h bei Raumtemperatur gerührt. Anschließend wird abfiltriert und das Filtrat von Lösungsmittel befreit. Der Filterkuchen wird mit 3×50 ml Methanol verrührt, vom Aluminiumoxid abfiltriert und das Filtrat im Vakuum bis zur Trockene eingeengt. Das ursprüngliche Filtrat wird mit dem Filtrat der Methanolausrührung vereinigt, zur Trockene eingeengt und anschließend an Kieselgel gereinigt (Laufmittel: Methylenchlorid/Methanol = 95 : 5). Nach Umkristallisation aus Acetonitril erhält man die Titelverbindung vom Schmp. 188 - 190°C.

### 2. 8-(2-(trans)-Hydroxy-2,3-dihydro-1-indenyloxy)-2,3-dimethyl-imidazo[1,2-a)pyridin

Die Titelverbindung vom Schmp. 182-183°C wird durch Umsetzung von 2,3-Dimethyl-8-hydroxy-imidazo[1,2-a] pyridin mit 1,2-Indenoxid nach der in Beispiel 1 angegebenen Arbeitsweise und Umkristallisation aus Diethylether erhalten

### 3. 8-(2-(trans)-Hydroxy-2,3-dihydro-1-indenyloxy)-2-methyl-imidazo[1,2-a)pyridin

Die Titelverbindung vom Schmp. 156-157°C wird durch Umsetzung von 8-Hydroxy-2-methyl-imidazo[1,2-a]pyridin mit 1,2-Indenoxid nach der in Beispiel 1 angegebenen Arbeitsweise erhalten.

### 4. 3-Cyanomethyl-8-(2-(trans)-hydroxy-2,3-dihydro-1-indenyloxy)-2-methyl-imidazo[1,2-a]pyridin

Die Titelverbindung vom Schmp. 181-183°C wird durch Umsetzung von 3-Cyanomethyl-8-hydroxy-2-methyl-imi- dazo[1,2-a]pyridin mit 1,2-Indenoxid nach der in Beispiel 1 angegebenen Arbeitsweise erhalten.

### 5. 8-(2-(trans)-Hydroxy-1,2,3,4-tetrahydro-1-naphthyloxy)-2,3-dimethylimidazo[1,2-a]pyridin

Die Titelverbindung wird als zähflüssiges Öl durch Umsetzung von 2,3-Dimethyl-8-hydroxy-imidazo[1,2-a]pyridin mit 1,2,3,4-Tetrahydronaphthatin-1,2-oxid nach der in Beispiel 1 angegebenen Arbeitsweise erhalten.

### 6. 3-Cyanomethyl-8-(2-(trans)-hydroxy-1,2,3,4-tetrahydro-1-naphthyloxy)-2-methyl-imidazo[1,2-a]pyridin

Die Titelverbindung vom Schmp. 154-155°C wird durch Umsetzung von 3-Cyanomethyl-8-hydroxy-2-methyl-imi- dazo[1,2-a]pyridin mit 1,2,3,4-Tetrahydronaphthatin-1,2-oxid nach der in Beispiel 1 angegebenen Arbeitsweise erhalten.

### 7. 3-Formyl-8-(2-(trans)-hydroxy-2,3-dihydro-1-indenyloxy)-2-methyl-imidazo[1,2-a]pyridin

Die Titelverbindung vom Schmp. 200-202°C wird durch Umsetzung von 3-Formyl-8-hydroxy-2-methyl-imidazo [1,2-a]pyridin mit 1,2-Indenoxid nach der in Beispiel 1 angegebenen Arbeitsweise erhalten.

### 8. 3-Hydroxymethyl-8-(2-(trans)-hydroxy-2,3-dihydro-1-indenyloxy)-2-methylimidazo[1,2-a]pyridin

1 g 3-Formyl-8-(2-(trans)-hydroxy-2,3-dihydro-1-indenyloxy)-2-methyl-imidazo[1,2-a]pyridin, gelöst in 150 ml Methanol, wird bei Raumtemperatur portionsweise mit überschüssigem Natriumborhydrid versetzt. Nach beendigter Reduktion wird mit 150 ml Eiswasser versetzt. Am Rotationsverdampfer werden im Vakuum die leichtflüchtigen Bestandteile abgezogen. Anschließend wird dreimal mit je 40 ml Ethylacetat extrahiert. Die vereinigten Extrakte werden im Vakuum vom Lösungsmittel befreit, und der kristalline Rückstand wird aus Methanol umkristallisiert. Man erhält die Titelverbindung vom Schmp. 192-193°C.

### 9. 8-(2-(trans)-Hydroxy-2,3-dihydro-1-indenyloxy)-2-methyl-3-nitroso-imidazo[1,2-a]pyridin

0,5 g 8-(2-(trans)-Hydroxy-2,3-dihydro-1-indenyloxy)-2-methyl-imidazo[1,2-a]pyridin; gelöst in 50 ml Tetrahydrofuran, werden bei 50°C mit 0,6 g käuflichem Butylnitrit versetzt und über Nacht bei dieser Temperatur gehalten. Nach Abziehen der flüchtigen Bestandteile im Vakuum wird der Rückstand mit wenig kaltem Acetonitril gewaschen und nach Abgießen des Lösungsmittels aus Acetonitril umkristallisiert. Man erhält 0,35 g gelbe Titelverbindung vom Schmp. 178-179°C.

### 10. 3-Amino-8-(2-(trans)-hydroxy-2, 3-dihydro-1-indenyloxy)-2-methyl-imidazo[1,2-a]pyridin

0,5 g 8-(2-(trans)-hydroxy-2,3-dihydro-1-indenyloxy)-2-methyl-3-nitroso-imidazo[1,2-a]pyridin werden in einer Mischung aus 10 ml Eisessig und 3 ml Wasser gelöst und bei 0°C Badtemperatur mit 0,5 g käuflichem Zinkpulverversetzt. Nach einer Stunde wird mit 100 ml Eiswasser verdünnt, mit gesättigter Natriumhydrogencarbonat-Lösung auf pH 8 eingestellt und mit Ethylacetat extrahiert. Die vereinigten Extrakte werden über Kaliumcarbonat getrocknet. Nach Abziehen des Lösungsmittels im Vakuum wird an Kieselgel gereinigt (Methylenchlorid/Methanol = 98: 2). Nach Umkristallisation aus Ethanol erhält man die Titelverbindung vom Schmp. 125-127°C.

### 11. 3-Amino-8-(2-(trans)-hydroxy-2,3-dihydro-1-indenyloxy)-2-methyl-imidazo[1,2-a]pyridin-Hydrochlorid

0,8 g 3-Amino-8-(2-(trans)-hydroxy-2,3-dihydro-[-indenyloxy)-2-methyl-imidazo[1,2-a]pyridin werden in 25 ml Isopropanol heiß gelöst und bei 20°C mit überschüssiger gesättigter etherischer Salzsäure versetzt. Die ausfallende Titelverbindung wird abgesaugt. Schmp. 145-147°C.

### 12. 8-(2-(trans)-Hydroxy-1,2,3,4-tetrahydro-1-naphthyloxy)-2-methyl-imidazo[1,2-a]pyridin

Die Titelverbindung vom Schmp. 196-197°C wird durch Umsetzung von 8-Hydroxy-2-methyl-imidazo[1,2-a]pyridin mit 1,2,3,4-Tetrahydronaphthalin-1,2-oxid nach der in Beispiel 1 angegebenen Arbeitsweise und Umkristallisation aus Acetonitril erhalten.

### 13. 3-Formyl-8-(2-(trans)-hydroxy-1,2,3,4-tetrahydro-1-naphthyloxy)-2-methylimidazo[1,2-alpyridin

Die Titelverbindung vom Schmp. 183-184°C wird durch Umsetzung von 3-Formyl-8-hydroxy-2-methyl-imidazo [1,2-a]pyridin mit 1,2,3,4-Tetrahydronaphthalin-1,2-oxid nach der in Beispiel 1 angegebenen Arbeitsweise und Umkristallisation aus Isopropanol erhalten.

### 14. 3-Hydroxymethyl-8-(2-(trans)-hydroxy-1,2,3,4-tetrahydro-1-naphthyloxy)-2-methyl-imidazol 1,2-alpyridin

Analog Beipiel 8 erhält man die Titelverbindung vom Schmp. 106-108°C durch Reduktion von 3-Formyl-8-(2-(trans)-hydroxy-1,2,3,4-tetrahydro-1-naphthyloxy)-2-methyl-imidazo[1,2-a]pyridin mit Natriumborhydrid.

### 15. 8-(2-(trans)-Hydroxy-1,2,3,4-tefrahydro-1-naphthyloxy)-2-methyl-3-nitroso-imidazo[1.2-alpyridin

Nitrosierung von 8-(2-(trans)-Hydroxy-1,2,3,4-tetranydro-1-naphthyloxy)-2-methyl-imidazo[1,2-a]pyridin analog Beispiel 9 liefert die Titelverbindung vom Schmp. 138-140°C.

### 16. 3-Formyl-8-(2-(trans)-hydroxy-2,3-dihydro-1-indenylamino)-2-methyl-imidazo[1,2-a]pyridin

Durch Umsetzung von 8-Amino-3-formyl-2-methyl-imidazo[1,2-a]pyridin mit 1,2-Indenoxid nach der in Beispiel 1 angegebenen Arbeitsweise erhält man die Titelverbindung vom Schmp. 236-238°C.

### 17. 3-Hydroxymethyl-8-(2-(trans)-hydroxy-2,3-dihydro-1-indenylamino)-2-methyl-imidazo[1,2-a]pyridin

Analog Beispiel 8 erhält man die Titelverbindung vom Schmp. 198-200°C durch Reduktion von 3-Formyl-8-(2-(trans)-hydroxy-2,3-dihydro-1-indenylamino)-2-methylimidazo[1,2-a]pyridin mit Natriumborhydrid.

### 18. 3-Amino-8-(2-(trans)-hydroxy-1,2,3,4-tetrahydro-1-naphthyloxy)-2-methylimidazo[1,2-a]pyridin-Hydrochlorid

Zur Lösung von 2,0 g 8-(2-(trans)-Hydroxy-1,2,3,4-tetrahydro-1-naphthyloxy)-2-methyl-3-nitroso-imidazo[1,2-a] pyridin in einer Mischung aus 20 ml Methanol und 20 ml bei Raumtemperatur gesättigter methanolischer Salzsäure wird bei 0°C unter kräftigem Rühren portionsweise ca. 1 g käufliches Raney-Nickel hinzugegeben. Nach 2 h wird abfiltriert, das Filtrat im Vakuum zur Trockene eingeengt und an Kieselgel gereinigt. Die Titelverbindung wird als zähflüssige Masse erhalten.

### 19. 3-Formyl-8-(2-(trans)-hydroxy-2.3-dihydro-1-indenyloxy)-2-methyl-imidazol 1,2-alpyridin

12 g (68 mmol) 3-Formyl-8-hydroxy-2-methyl-imidazo[1,2-a]pyridin, 18 g (136 mmol) 1,2-Indenoxid und 9,6 ml (68 mmol) Triethylamin werden in 95 ml Methanol/Wasser (4 : 1) 14 h bei 40°C gerührt. Man engt bei 50°C im Rotationsverdampfer ein, versetzt mit 200 ml Eis-Wasser, macht mit Natronlauge alkalisch (ca. pH 13) und filtriert den Niederschlag ab. Das Filtrat wird mit Dichlormethan ausgeschüttelt und die organische Phase auf 250 ml mit Dichlormethan verdünnt, mit dem Niederschlag vereinigt, mit Magnesiumsulfat getrocknet und mit Aktivkohle geklärt. Man versetzt mit 250 ml Toluol, destilliert Dichlormethan ab, klärt in der Siedehitze mit Bleicherde (z.B. Tonsil^{®}), engt auf die Hälfte ein und läßt im Kühlschrank kristallisieren. Man erhält 17,6 g (84 %) der Titelverbindung Schmp. 200 - 202°C.

### 20. 3-Formyl-8-(2-(trans)-hydroxy-2.3-dihydro-1-indenyloxy)-2-methyl-imidazo[1,2-a]pyridin

4,4 g (25 mmol) 3-Formyl-8-hydroxy-2-methyl-imidazo[1,2-a]pyridin, 10,7 g (50 mmol) (trans)-2-Brom-1-indanol und 13,8 g Kaliumcarbonat werden in 120 ml Methanol/Wasser (4: 1) 16 h bei 40°C gerührt. Man arbeitet analog Beispiel 19 auf und erhält 4,8 g (62 %) der Titelverbindung. Schmp. 200 - 202°C (aus Methanol).

### 21. 3-Formyl-8-(2-(trans)-hydroxy-2,3-dihydro-1-indenyloxy)-2-methyl-imidazo[1,2-a]pyridin

Zu 85,2 g (400 mmol) (trans)-2-Brom-1-indanol in 600 ml Isopropylalkohol tropft man bei Raumtemperatur unter Rühren 39,7 ml 10 N Kalilauge und rührt noch 15 Min. bei Raumtemperatur Man destilliert den Isopropylalkohol bei 40°C / 5-8 mbar ab, versetzt mit 350 ml Methanol/Wasser (4 : 1 30,4 g Kaliumcarbonat und 35,2 g (200 mmol) 3-Formyl-83-Formyl-8-hydroxy-2-methyl-imidazo[1,2-a]pyridin und rührt noch 10 h bei 40°C und 8 h bei Raumtemperatur. Man arbeitet analog Beispiel 19 auf und erhält 47,2 g (76,5 %) der Titelverbindung. Schmp. 200 - 202°C (aus Methanol).

### 22. 6-Chlor-3-formyl-8-(2-(trans)-hydroxy-2, 3-dihydro-1-indenyloxy)-2-methylimidazo[1,2-a]pyridin

Die Titelverbindung vom Schmp. 220 - 222°C wird durch Umsetzung von 6-Chlor-3-formyl-8-hydroxy-2-methyl- imidazo[1,2-a]pyridin mit 1,2-Indenoxid nach der in Beispiel 19 angegebenen Arbeitsweise und Umkristallisation aus Methanol erhalten.

### 23. 6-Chlor-3-hydroxymethyl-8-(2-(trans)-hydroxy-2,3-dihydro-1-indenyloxy)-2-methyl-imidazo[1,2-a]pyridin

Analog Beispiel 8 erhält man die Titelverbindung vom Schmp. 210°C (unter Zersetzung) durch Reduktion von 6-Chlor-3-formyl-8-(2-(trans)-hydroxy-2,3-dihydro-1-indenyloxy)-2-methyl-imidazo[1,2-a]pyridin mit Natriumborhydrid und Umkristallisation aus Methanol.

### 24. 6-Chlor-8-(2-(trans)-hydroxy-2,3-dihydro-1-indenyloxy)-2-methyl-imidazo[1,2-a]pyridin

Die Titelverbindung vom Schmp 175 - 177°C wird durch Umsetzung von 6-Chlor-8-hydroxy-2-methyl-imidazo[i, 2-a]pyridin mit 1,2-Indenoxid nach der in Beispiel 19 angegebenen Arbeitsweise und Umkristallisation aus Ethylacetat erhalten.

### 25. 6-Chlor-8-(2-(trans)-hydroxy-2,3-dihydro-1-indenyloxy)-2-methyl-3-nitrosoimidazol 1,2-alpyridin

Nitrosierung von 6-Chlor-8-(2-(trans)-hydroxy-2,3-dihydro-1-indenyloxy)-2-methyl-imidazo[1,2-a]pyridin analog Beispiel 9 liefert die Titelverbindung.

### 26. 3-Amino-6-chlor-8-(2-(trans)-hydroxy-2,3-dihydro-1-indenyloxy)-2-methylimidazol 1,2-alpyridin

Die Titelverbindung vom Schmp 223 - 225°C wird analog Beispiel 10 durch Reduktion von 6-Chlor-B-(2-(trans)-hydroxy-2,3-dihydro-1 -indenyloxy)-2-methyl-3-nitroso-imidazo[1,2-a]pyridin mit Zink in wässriger Essigsäure und Reinigung an Kieselgel (Methylenchlorid/Methanol = 95 : 5 als Elutionsgemisch) erhalten.

### 27. 8-(5-Fluor-2-(trans)-hydroxy-2,3-dihydro-1-indenyloxy)-3-formyl-2-methylimidazo[1,2-a]pyridin

Die Titelverbindung vom Schmp. 205°C wird durch Umsetzung von 3-Formyl-8-hydroxy-2-methyl-imidazo[1,2-a] pyridin mit 5-Fluor-inden-1,2-oxid nach der in Beispiel 20 angegebenen Arbeitsweise und Umkristallisation aus Methanol erhalten.

### 28. 8-(5-Fluor-2-(trans)-hydroxy-2,3-dihydro-1-indenyloxy)-3-hydroxymethyl-2-methyl-imidazo[1,2-a]pyridin

Analog Beispiel 8 erhält man die Titelverbindung vom Schmp. 197 - 199°C durch Reduktion von 8-(5-Fluor-2-(trans)-hydroxy-2,3-dihydro-1-indenyloxy)-3-formyl-2-methyl-imidazo[1,2-a]pyridin mit Natriumborhydrid und Umkristallisation aus Ethanol.

### 29. 8-(5-Fluor-2-(trans)-hydroxy-2,3-dihydro-1-indenyloxy)-2-methyl-imidazo[1,2-a]pyridin

Die Titelverbindung vom Schmp. 174-175°C wird durch Umsetzung von 8-Hydroxy-2-methyl-imidazo[1,2-a]pyridin mit 5-Fluor-inden-1,2-oxid nach der in Beispiel 20 angegebenen Arbeitsweise und Umkristallisation aus Acetonitril erhalten.

### 30. 8-(5-Fluor-2-(trans)-hydroxy-2,3-dihydro-1-indenyloxy)-2-methyl-3-nitrosoimidazo[1,2-a]pyridin

Nitrosierung von 8-(5-Fluor-2-(trans)-hydroxy-2,3-dihydro-1-indenyloxy)-2-methyl-imidazo[1,2-a]pyridin analog Beispiel 9 liefert nach Umkristallisation aus Acetonitril die Titelverbindung vom Schmp. 150°C (unter Zers.).

### 31. 3-Amino-8-(5-fluor-2-(trans)-hydroxy-2, 3-dihydro-1-indenyloxy)-2-methylimidazo[1,2-a]pyridin-Hydrochlorid

Die Titelverbindung vom Schmp. 168 - 170°C wird analog Beispiel 10 durch Reduktion von 8-(5-Fluor-2-(trans)-hydroxy-2,3-dihydro-1-indenyloxy)-2-methyl-3-nitroso-imidazo[1,2-a]pyridin mit Zink in wässriger Essigsäure und Fällung des Hydrochlorids aus einer 2-Propanol-Lösung mit Hilfe von HCI/Ether-Lösung erhalten.

### 32. (1'R,2'R)-3-Formyl-8-(2-hydroxy-2,3-dihydro-1-indenyloxy)-2-methyl-imidazo[1,2-a]pyridin

Die Titelverbindung vom Schmp. 200 - 202°C wird durch Umsetzung von 3-Formyl-8-hydroxy-2-methyl-imidazo [1,2-a]pyridin mit (+)-(IS,2R)-Indenoxid nach der in Beispiel 19 angegebenen Arbeitsweise und Reinigung an Kieselgel (Methylenchlorid/Methanol = 95 : 5 als Elutionsgemisch) erhalten. [α]=²²_{D} - 122,2° (c = 1, Chloroform).

### 33. (-)-(1'R,2'R)-3-Hydroxymethyl-8-(2-hydroxy-2,3-dihydro-1-indenyloxy)-2-methyl-imidazol 1,2-alpyridin

Analog Beispiel 8 erhält man die Titelverbindung vom Schmp. 198 - 200°C ([α]²⁰_{D} = -100°, Methylenchlorid/Methanol = 1 : 1) durch Reduktion von (1'R,2'R)-3-Formyl-8-(2-hydroxy-2,3-dihydro-1-indenyloxy)-2-methyl-imidazo[1,2-a] pyridin mit Natriumborhydrid.

### 34. (-)-(1'R,2'R)-3-Cyanomethyl-8-(2-hydroxy-2,3-dihydro-1-indenyloxy)-2-methyl-imidazo[1,2-a]pyridin

Die Titelverbindung vom Schmp. 173 - 174°C ([α]²²_{D} = -219°, Chloroform) wird durch Umsetzung von 3-Cyanomethyl-8-hydroxy-2-methyl-imidazo[1,2-a]pyridin mit (+)-(1 S,2R)-Indenoxid nach der in Beispiel 19 angegebenen Arbeitsweise und Waschen mit kaltem Methanol erhalten.

### 35. (1'R,2'R)-8(2-Hydroxy-2,3-dihydro-1-indenyloxy)-2-methyl-imidazo[1,2-a]pyridin

Die Titelverbindung wird als amorpher Festkörper durch Umsetzung von 8-Hydroxy-2-methyl-imidazo[1,2-a]pyridin mit (+)-(1 S,2R)-Indenoxid nach der in Beispiel 19 angegebenen Arbeitsweise und Reinigung an Kieselgel (Methylenchlorid/Methanol = 95: 5 als Elutionsgemisch) erhalten.

### 36. (1'R,2'R)-8-(2-Hydroxy-2,3-dihydro-1-indenyloxy)-2-methyl-3-nitroso-imidazo[1,2-a]pyridin

Nitrosierung von (1'R,2'R)-8-(2-Hydroxy-2,3-dihydro-1-indenyloxy)-2-methyl-imidazo[1,2-a]pyridin analog Beispiel 9 liefert die Titelverbindung nach Reinigung an Kieselgel (Methylenchlorid/Methanol = 95 : 5 als Elutionsgemisch) als grünes amorphes Pulver.

### 37. (+)-(1'R,2'R)-3-Amino-8-(2-hydroxy-2,3-dihydro-1-indenyloxy)-2-methyl-imidazo[1,2-a]pyridin-Hydrochlorid

Die Titelverbindung vom Schmp. 151 - 152°C ([α]²⁰_{D} = +31 ° , Methanol) wird analog Beispiel 10 durch Reduktion von (1'R,2'R)-8-(2-Hydroxy-2,3-dihydro-1-indenyloxy)-2-methyl-3-nitroso-imidazo[1,2-a]pyridin mit Zink in wässriger Essigsäure und Fällung des Hydrochlorids aus einer 2-Propanol-Lösung mit Hilfe von HCI/Ether-Lösung erhalten.

### 38. (+)-(1S,2S)-3-Cyanmethyl-2-methyl-8-(2-hydroxy-2,3-dihydro-1-indenyloxy)imidazo[1.2-a]pyridin

750 mg 3-Cyanmethyl-8-hydroxy-2-methyl-imidazo[1,2-a]pyridin werden nach der in Beispiel 19 angegebenen Arbeitsweise mit 210 mg (-)-1 R,2S-Indenoxid und 400 mg Triethylamin in wässrigem Methanol (60 ml Methanol + 15 ml Wasser) bei Raumtemperatur umgesetzt. Nach 3 Tagen wird die Lösung eingeengt und der Rückstand in 50 ml Wasser aufgenommen. Der Niederschlag wird abfiltriert und anschließend durch Chromatographie an Kieselgel (Fließmittel: Essigester/Methanol = 10: 1) gereinigt. Nach Einengen wird der Rückstand aus Methylenchlorid/Cyclohexan ausgefällt. 120 mg der Titelverbindung werden isoliert. [α]²²_{D}+ 113° (c = 1, Chloroform).

### 39. (+)-(1S,2S)-3-Formyl-2-methyl-8-(2-hydroxy-2,3-dihydro-1-indenyloxy)-imidazo[1,2-a]pyridin

3,30 g 3-Formyl-8-hydroxy-2-methyl-imidazo[1,2-a]pyridin und 1,40 g (-)-1 R,2S-lndenoxid werden nach der in Beispiel 19 angegebenen Arbeitsweise mit 1,90 g Triethylamin in 425 ml wässrigem Methanol (350 ml Methanol + 75 ml Wasser) bei 50°C umgesetzt. Nach 8 h wird eingeengt und der Rückstand in 150 ml Wasser aufgenommen. Nach Zugabe von 4 g Natriumcarbonat wird mit 4 x 100 ml Essigester extrahiert. Die organischen Phasen werden mit 2 x 200 ml Natriumcarbonatlösung und 4 x 200 ml Wasser gewaschen, über Magnesiumsulfat getrocknet und eingeengt. Der feste Rückstand wird in 100 ml Cyclohexan aufgenommen, filtriert und getrocknet. 1,51 g der Titelverbindung vom Schmp. 187 - 192°C werden isoliert. [α]²²_{D} = +112° (c = 0,₅, Chloroform).

### 40. (+)-(1S,2S)-3-Hydroxymethyl-2-methyl-8-(2-hydroxy-2,3-dihydro-1-indenyloxy)-imidazo[1,2-a]pyridin

Eine Lösung von 1,39 g (+)-(1S,2S)-3-Formyl-2-methyl-imidazo[1,2-a]pyridin in 300 ml Methanol wird bei 0°C mit 350 mg Natriumborhydrid versetzt Nach Erwärmen auf Raumtemperatur wird noch 1 h gerührt und anschließend mit 100 ml Wasser versetzt. Das Methanol wird am Rotationsverdampfer abgezogen. Nach Zugabe von 150 ml Wasser wird auf +4°C gekühlt und abgesaugt. Der noch feuchte Rückstand wird in 300 ml Aceton aufgenommen, filtriert und mit Diisopropylether nachgewaschen. Nach Trocknen im Hochvakuum werden 960 mg der Titelverbindung vom Schmp. 188 - 192°C isoliert. [α]²²_{D} +105° (c = 0,5, Chloroform/Methanol = 1 : 1).

### 41. (+)-(1S,2S)-3-Formyl-2-methyl-8-[2-hydroxy-1,2,3,4-tetrahydro-1-naphthyloxy)-imidazo[1,2-a]pyridin

580 mg 3-Formyl-8-hydroxy-2-methyl-imidazo[1,2-a]pyridin und 580 mg (+)-1 R,2S-1,2,3,4-Tetrahydro-naphthalin-1,2-oxid werden nach der in Beispiel 19 angegebenen Arbeitsweise mit 340 mg Triethylamin in 43 ml wässrigem Methanol (35 ml Methanol + 8 ml Wasser) bei 60°C umgesetzt. Nach 8 h wird, wie in Beispiel 41 beschrieben, aufgearbeitet. Nach Ausrühren aus Petrolether und Trocknen im Hochvakuum werden 250 mg der Titelverbindung isoliert [α]²²_{D} = +38° (c = ₁, Chloroform). D

### 42. (+)-(1S,2S)-3-Hydroxymethyl-2-methyl-8-(2-hydroxy-1,2,3,4-tetrahydro-1-naphthyloxy)-imidazo[1,2-a]pyridin

Eine Lösung von 200 mg (-)-(1S,2S)-3-Formyl-2-methyl-8-(2-hydroxy-1,2,3,4-tetrahydro-1-naphthyloxy)-imidazo [1,2-a]pyridin in 75 ml Methanol wird, wie in Beispiel 8 beschrieben, mit 80 mg Natriumborhydrid 4 h bei Raumtemperatur umgesetzt. Nach Zugabe von 100 ml Wasser wird das Methanol am Rotationsverdampfer abdestilliert. Der im wässrigen Rückstand ausgefallene Niederschlag wird abfiltriert, mit Wasser gewaschen und im Hochvakuum getrocknet. 170 mg der Titelverbindung vom Schmp. 102 - 104°C werden isoliert. [α]²²_{D} = +90° (c = 0,5, Chloroform).

### Ausgangsverbindungen

### A. 8-Amino-2-methyl-imidazo[1,2-a]pyridin-Hydrobromid

Eine Lösung von 16,5 g 2-Methyl-B-nitro-imidazo[1,2-a]pyridin-Hydrobromid in 800 ml Methanol wird nach Zusatz einer katalytischen Menge käuflichen Pd/C-Katalysators bei Raumtemperatur unter geringem Überdruck über 8 h mit Wasserstoff gesättigt. Danach wird der Katalysator abfiltriert und das Filtrat zur Trockene eingeengt. Nach Umkristallisation aus Acetonitril werden 11,9 g der Titelverbindung vom Schmp. 237-238°C erhalten.

### B. 8-Amino-2,3-dimethyl-imidazo[1,2-a]pyridin

Analog Beispiel A werden 11,3 g der Titelverbindung vom Schmp. 148-150°C durch katalytische Reduktion von 19,0 g 2,3-Dimethyl-8-nitro-imidazo[1,2-a]pyridin, gelöst in 700 ml Methanol, erhalten.

### C 2-Methyl-8-nitro-imidazor1,2-alpyridin-Hydrobromid

20,0 g 2-Amino-3-nitropyridin und 22 g Bromaceton werden in 1 1 Ethanol 55 h am Rückfluß gekocht. Anschließend wird im Eisbad abgekühlt, das kristalline Hydrobromid abgesaugt und mit Ethanol gewaschen. Nach Trocknung im Vakuum bei 50°C erhält man 17,2 g der Titelverbindung vom Schmp. 300°C (Zersetzung).

### D. 3-Formyl-2-methyl-8-nitro-imidazo[1,2-a]pyridin

Zur Lösung von 7,6 g 2-Methyl-8-nitro-imidazo[1,2-a]pyridin in 100 ml Dimethylformamid werden bei 0°C Innentemperatur 20 ml Phosphoroxychlorid innerhalb von 5 Min. zugetropft. Nach einstündigem Rühren bei 0°C und zweistündigem Rühren bei 70°C wird erneut auf 0 C abgekühlt. Nach Zutropfen von 10 ml Phosphoroxychlorid wird in der gleichen Abfolge wie zuvor verfahren Nach beendeter Umsetzung wird auf 200 ml Eiswasser gegossen, mit gesättigter Natriumhydrogencarbonatlösung neutralisiert und dreimal mit je 50 ml Ethylacetat extrahiert.

Nach Abziehen des Lösungsmittels im Vakuum wird der verbliebene Rückstand mit Ether gewaschen und aus Acetonitril umkristallisiert, wobei die Titelverbindung vom Schmp. 188-189°C erhalten wird.

### E. 8-Amino-3-formyl-2-methyl-imidazo[1,2-a]pyridin

Eine Lösung von 0,5 g 3-Formyl-2-methyl-8-nitro-imidazo[1,2-a]pyridin in 200 ml Methanol wird unter Zusatz einer katalytischen Menge käuflichen Pd/C-Katalysators bei Raumtemperatur unter geringem Überdruck 1,5 h mit Wasserstoff gesättigt. Der Katalysator wird danach abfiltriert, das Filtrat im Vakuum bis zur Trockene eingeengt und der resultierende kristalline Rückstand aus Acetonitril umkristallisiert, wobei die Titelverbindung vom Schmp. 146-147°C erhalten wird

### F. 6,8-Dichlor-2-methyl-imidazo[1,2-a]pyridin

Ein Gemisch aus 30 g 2-Amino-3,5-dichlorpyridin und 39 ml Chloraceton in 260 ml Ethanol wird 64 h unter Rückfluß erhitzt; die flüchtigen Anteile werden im Vakuum abgezogen, und der Rückstand wird in 200 ml Wasser suspendiert. Nach Einstellung eines pH-Wertes von 7 - 8 mittels gesättigter wässriger NaHC0₃-Lösung wird abgesaugt, der Filterkuchen im Vakuum bis zur Gewichtskonstanz getrocknet und aus Cyclohexan umkristallisiert. Es werden 17,8 g der Titelverbindung vom Schmp. 156 -158°C erhalten.

### G. 8-Benzyloxy-6-chlor-2-methyl-imidazo[1,2-a]pyridin-Hydrochlorid

Zu einer Lösung von 5,4 ml Benzylalkohol in 300 ml trockenem Dimethylformamid werden bei Raumtemperatur 1,8 g käufliches 80 %iges Natriumhydrid in Paraffin in kleinen Portionen zugegeben. Die entstandene Benzylat-Lösung wird noch weitere 2 h gerührt. Anschließend wird auf - 40°C abgekühlt, mit 10,0 g 6_{;}8-Dichlor-2-methyl-imidazo[1,2-a] pyridin versetzt und weitere 4 h gerührt. Man läßt die Temperatur langsam auf 12°C steigen und rührt bei dieser Temperatur 48 h. Danach wird auf 1,5 kg Eiswasser gegossen, die Lösung mit 1 N-HCI auf pH = 7 eingestellt und dreimal mit je 100 ml Ethylacetat extrahiert. Die vereinigten Extrakte werden über Natriumsulfat getrocknet und am Rotationsverdampfer bis zur Trockene eingeengt. Man nimmt danach in 100 ml 2-Propanol auf und fällt die Titelverbindung durch Zusatz von gesättigter HCI-Ether-Lösung. Nach dem Absaugen des Niederschlages wird zweimal aus Isopropanol umkristallisiert, wobei 4,5 g der Titelverbindung vom Schmp. 180 - 182°C erhalten werden.

### H 8-Benzyloxy-6-chlor-3-formyl-2-methyl-imidazo[1,2-a]pyridin

Die Titelverbindung vom Schmp. 137 - 138°C (aus Acetonitril) wird analog Beispiel D aus 8-Benzyloxy-6-chlor-2-methyl-imidazo[1,2-a]pyridin und Phosphoroxychlorid/Dimethylformamid erhalten.

### 1. 6-Chlor-3-formyl-8-hydroxy-2-methyl-imidazo[1,2-a]pyridin

Ein Gemisch aus 0,5 g 8-Benzyloxy-6-chlor-3-formyl-2-methyl-imidazo[1,2-a]pyridin, 2,5 ml Essigsäure und 2,5 ml 48 %iger wässrige Bromwasserstoff-Lösung wird 2 h unter Rückfluß erhitzt. Anschließend wird nach Abkühlen auf Raumtemperatur auf 50 ml Eiswasser gegossen, mit 1 N-Natronlauge neutralisiert und dreimal mit je 20 ml Ethylacetat extrahiert. Nach Trocknung der vereinigten Extrakte über Natriumsulfat wird das Lösungsmittel abgezogen und der verbleibende feste Rückstand aus Dioxan umkristallisiert. Es werden 0,2 g der Titelverbindung vom Schmp. 256°C (unter Zersetzung) erhalten.

### J. 6-Chlor-8-hydroxy-2-methyl-imidazor1 ,2-alpyridin

Die Titelverbindung vom Schmp. 232°C (unter Zersetzung) wird analog Beispiel 1 durch Behandlung von 8-Ben- zyloxy-6-chlor-2-methyl-imidazo[1,2-a]pyridin mit Essigäure/Bromwasserstoff-Lösung nach Reinigung an Kieselgel (Ethylacetat/Petrolether = 6/4 als Elutionsgemisch) erhalten.

### K. 3-Formyl-2-methyl-8-(2-(trans)-methylsulfonyloxy-2,3-dihydro-1-indenyloxy)-imidazo[1,2-alpyridin

In die Suspension von 10 g 3-Formyl-8-(2-(trans)-hydroxy-2,3-dihydro-1-indenyloxy)-2-methyl-imidazo[1,2-a]pyridin in 200 ml trockenem Dichlormethan gibt man unter Rühren und Feuchtigkeitsausschluß 8,5 ml Triethylamin, kühlt im Eisbad ab und tropft eine Lösung von 5 ml Methansulfonsäurechlorid in 10 ml trockenem Dichlormethan zu. Man rührt 1 h unter Eiskühlung nach, gießt die gebildete Lösung auf Eiswasser und extrahiert mehrmals mit Dichlormethan. Die gesammelten organischen Phasen werden mit Wasser gewaschen, getrocknet und das Lösungsmittel im Vakuum abgezogen. Der gelbliche Rückstand wird mit Diethylether ausgerührt, abgesaugt und getrocknet. Man erhält 12 g der Titelverbindung vom Schmp. 158 - 160°C. - Analog erhält man 2-Methyl-8-(2-(trans)-methylsulfonyloxy-2,3-dihydro-1-indenyloxy)-imidazo[1,2-a]pyridin aus 2-Methyl-8-(2-(trans)-hydroxy-2,3-dihydro-1-indenyloxy)-imidazo[1,2-a]pyridin und Methansulfonsäurechlorid. Schmp. 122 - 125°C (Zersetzung).

### Gewerbliche Anwendbarkeit

Die Verbindungen der Formel 1 und ihre Salze besitzen wertvolle pharmakologische Eigenschaften, die sie gewerblich verwertbar machen. Sie weisen insbesondere eine ausgezeichnete Magen- und Darmschutzwirkung bei Warmblütern auf. Darüberhinaus zeichnen sich die erfindungsgemäßen Verbindungen durch eine hohe Wirkungsselektivität, das Fehlen wesentlicher Nebenwirkungen und eine große therapeutische Breite aus.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. bestimmte Antiphlogistika und Antirheumatika), Chemikalien (z. B. Ethanol), Magensäure oder Streßsituationen verursacht werden können.

In ihren ausgezeichneten Eigenschaften erweisen sich die erfindungsgemäßen Verbindungen an verschiedenen Modellen, in denen die antiulcerogenen und die antisekretorischen Eigenschaften bestimmt werden, überraschenderweise den aus dem Stand der Technik bekannten Verbindungen deutlich überlegen. Aufgrund dieser Eigenschaften sind die Verbindungen der Formel 1 und ihre pharmakologisch verträglichen Salze für den Einsatz in der Human-und Veterinärmedizin hervorragend geeignet, wobei sie insbesondere zur Behandlung und/oder Prophylaxe von Ulcuserkrankungen des Magens und/oder Darms verwendet werden.

Ein weiterer Gegenstand der Erfindung sind daher die erfindungsgemäßen Verbindungen zur Anwendung bei der Behandlung und/oder Prophylaxe der vorstehend genannten Krankheiten.

Ebenso umfaßt die Erfindung die Verwendung der erfindungsgemäßen Verbindungen zur Herstellung von Arzneimitteln, die zur Behandlung und/oder Prophylaxe der vorstehend genannten Krankheiten eingesetzt werden.

Weiterhin umfaßt die Erfindung die Verwendung der erfindungsgemäßen Verbindungen zur Behandlung und/oder Prophylaxe der vorstehend genannten Krankheiten.

Ein weiterer Gegenstand der Erfindung sind Arzneimittel, die ein oder mehrere Verbindungen der Formel I und/ oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfs- oder Trägerstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95% beträgt.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; Tranquilizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaesthetika, wie z.B. Tetracain, Procain; Antibiotika, wie Penicilline, Tetracycline etc. gegebenenfalls auch Fermente, Vitamine oder Aminosäuren enthalten.

Hervorzuheben ist in diesem Zusammenhang insbesondere die Kombination der erfindungsgemäßen Verbindungen mit Pharmaka, die die Säuresekretion hemmen, wie beispielsweise H₂-Blockern (z.B. Cimetidin, Ranitidin), mit sogenannten peripheren Anticholinergika (z B. Pirenzepin, Telenzepin, Zolenzepin) mit dem Ziel, die Hauptwirkung in additivem oder überadditivem Sinn zu verstärken und/oder die Nebenwirkungen zu eliminieren oder zu verringern, oder ferner mit antibakteriell wirksamen Substanzen (wie z.B. Cephalosporinen, Tetracyclinen, Nalidixinsäure, Penicillinen etc ) zur Bekämpfung von Campylobacter pyloridis.

### Pharmakologie

Die ausgezeichnete Magenschutzwirkung und die magensekretionshemmende Wirkung der erfindungsgemäßen Verbindungen kann in Untersuchungen an tierexperimentellen Modellen nachgewiesen werden. Die in der nachstehend aufgeführten Tabelle untersuchten erfindungsgemäßen Verbindungen sind mit Nummern versehen worden, die den Nummern der Beispiele entsprechen.

### Prüfung der antiulceroqenen und sekretionshemmenden Wirkung an der modifizierten Shay-Ratte

In der folgenden Tabelle ist der Einfluß der erfindungsgemäßen Verbindungen nach intraduodenaler (i. d.) Gabe auf die Läsionsbildung sowie die Magensäuresekretion bei der modifizierten Shay-Ratte dargestellt.

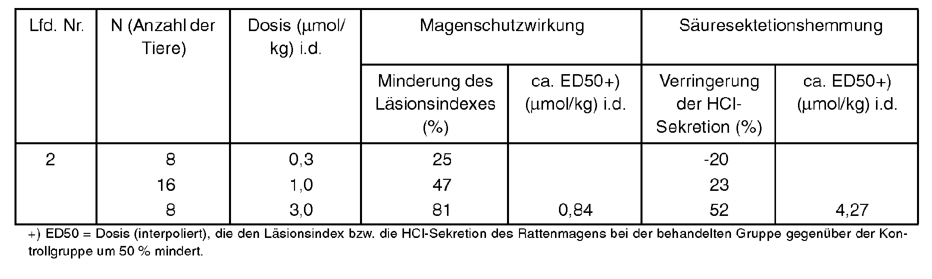

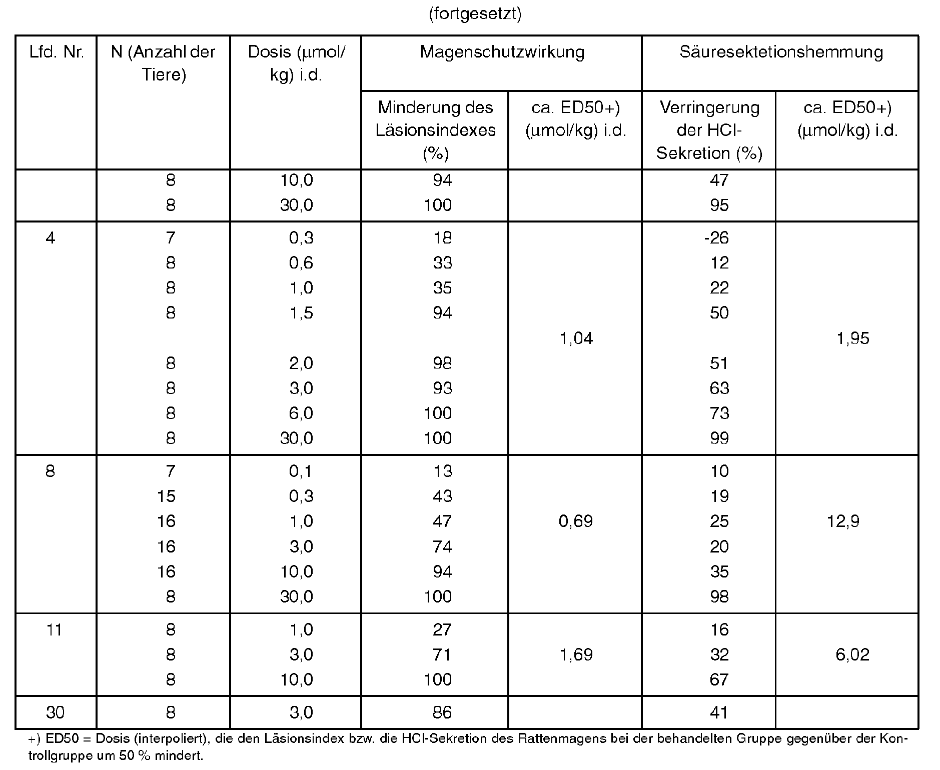

Methodik

Die Ulcusprovokation erfolgt an 24 Stunden nüchternen Ratten (weiblich, 180-200 g, 4 Tiere je Käfig auf hohem Gitterrost) durch Pylorusligatur (unter Diethylethernarkose) und orale Applikation von 100 mg/10ml/kg Acetylsalicylsäure. Die zu prüfenden Substanzen werden intraduodenal (2,5 ml/kg) unmittelbar nach der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens.

Der Magen wird längs der großen Kurvatur eröffnet und auf einer Korkplatte aufgespannt, nachdem zuvor die Menge des sezernierten Magensaftes (Volumen) und später sein HCI-Gehalt (Titration mit Natronlauge) bestimmt wird. Mit einem Stereomikroskop werden bei 10-facher Vergrößerung Anzahl und Größe (=Durchmesser) vorhandener Ulcera ermittelt. Das Produkt aus Schweregrad (gemäß nachfolgender Punkteskala) und Anzahl der Ulcera dient als individueller Läsionsindex.

Punkteskala:

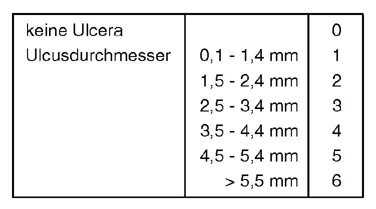

Als Maß für den antiulcerogenen Effekt dient die Minderung des mittleren Läsionsindex jeder behandelten Gruppe gegenüber dem der Kontrollgruppe (=100 %). Die ED50 bezeichnet diejenige Dosis, die den mittleren Läsionsindex bzw. die HCI-Sekretion gegenüber der Kontrolle um 50 % mindert.

## Patentansprüche (Patentansprüche für folgende(n) Vertragsstaat(en) : AT, BE, CH, DE, FR, GB, IT, LI, LU, NL, SE)

1. Verbindungen der Formel 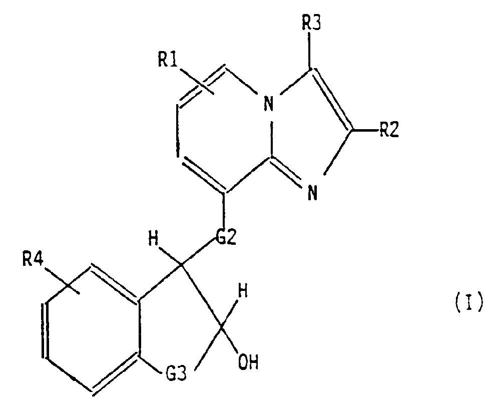 worin
R1 Wasserstoff (H) oder Halogen,
R2 1-4C-Alkyl,
R3 Wasserstoff (H), 1-4C-Alkyl, Formyl, Hydroxy-1-4C-alkyl, Cyano-1-4C-alkyl, Nitroso oder Amino, R4 Wasserstoff (H) oder Halogen,
G2 0 (Sauerstoff) oder NH und
G3 -CH₂- oder -CH₂CH₂- bedeutet,
wobei G2 und OH trans-ständig zueinander stehen, und ihre Salze.

2. Verbindungen nach Anspruch 1, worin
R1 Wasserstoff (H)
bedeutet.

3. Verbindungen nach Anspruch 1, worin
R1 Wasserstoff,
R2 Methyl,
R3 Wasserstoff, Methyl, Formyl, Hydroxymethyl, Cyanomethyl oder Amino und
R4 Wasserstoff, Chlor oder Fluor
bedeutet.

4. Verbindungen nach Anspruch 1, worin
R1 Wasserstoff,
R2 Methyl,
R3 Wasserstoff, Methyl, Hydroxymethyl, Cyanomethyl oder Amino und
R4 Wasserstoff
bedeutet.

5. Verbindungen nach Anspruch 1, worin
R1 Wasserstoff, Chlor oder Fluor,
R2 Methyl oder Ethyl,
R3 Wasserstoff, Methyl, Formyl, Hydroxymethyl, Cyanomethyl oder Amino und
R4 Wasserstoff, Chlor oder Fluor
bedeutet

6. Verbindungen der Formel nach Anspruch 1, worin
R1 Wasserstoff,
R2 Methyl,
R3 Wasserstoff, Methyl Hydroxymethyl, Cyanomethyl oder Amino und
R4 Wasserstoff oder Fluor bedeutet.

7. Verfahren zur Herstellung der Verbindungen der Formel nach Anspruch 1 und ihrer Salze, dadurch gekennzeichnet, daß man Imidazopyridine der Formel II mit Bicyclen der Formel III umsetzt, 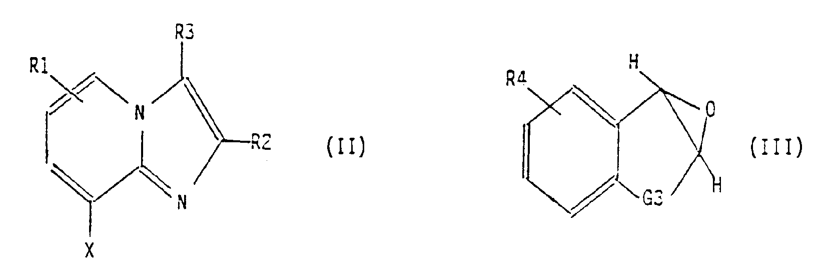 und gewünschtenfalls anschließend erhaltene Verbindungen der Formel 1, worin R3 die Bedeutung Formyl bzw. Nitroso hat, zu den Verbindungen der Formel worin R3 die Bedeutung Hydroxymethyl bzw. Amino hat, reduziert, und/oder gewünschtenfalls anschließend erhaltene Salze in die freien Verbindungen oder erhaltene freie Verbindungen in die Salze überführt, wobei die Ausgangsverbindungen als solche oder in Form ihrer Salze eingesetzt werden und wobei R1, R2, R3, R4 und G3 die in Anspruch 1 angegebenen Bedeutungen haben und X OH oder NH₂ bedeutet

8. Arzneimittel enthaltend eine oder mehrere Verbindungen nach einem oder mehreren der Ansprüche 1 bis 6 und/ oder ihre pharmakologisch verträglichen Salze.

9. Verbindungen nach einem oder mehreren der Ansprüche 1 bis 6 und ihre pharmakologisch verträglichen Salze zur Anwendung bei der Verhütung und Behandlung gastrointestinaler Krankheiten.

10. Verwendung von Verbindungen nach einem oder mehreren der Ansprüche 1 bis 6 und ihren pharmakologisch verträglichen Salzen zur Herstellung von Arzneimitteln für die Verhütung und Behandlung gastrointestinaler Krankheilen.

## Patentansprüche (Patentansprüche für folgende(n) Vertragsstaat(en) : ES, GR)

1. Verfahren zur Herstellung der Verbindungen der Formel 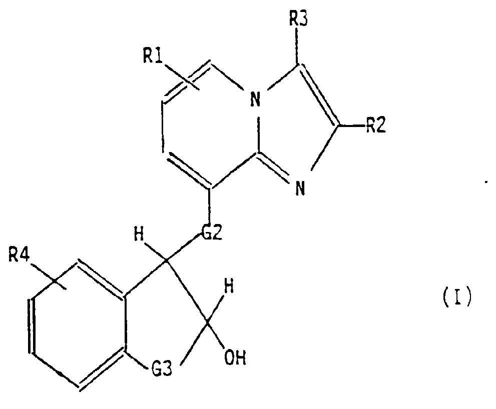 worin
R1 Wasserstoff (H) oder Halogen,
R2 1-4C-Alkyl,
R3 Wasserstoff (H), 1-4C-Alkyl, Formyl, Hydroxy-1-4C-alkyl, Cyano-1-4C-alkyl, Nitroso oder Amino,
R4 Wasserstoff (H) oder Halogen,
G2 0 (Sauerstoff) oder NH und
G3 -CH₂- oder -CH₂CH₂- bedeutet,
wobei G2 und OH trans-ständig zueinander stehen,
und ihrer Salze, dadurch gekennzeichnet, daß man Imidazopyridine der Formel II mit Bicyclen der Formel III umsetzt, 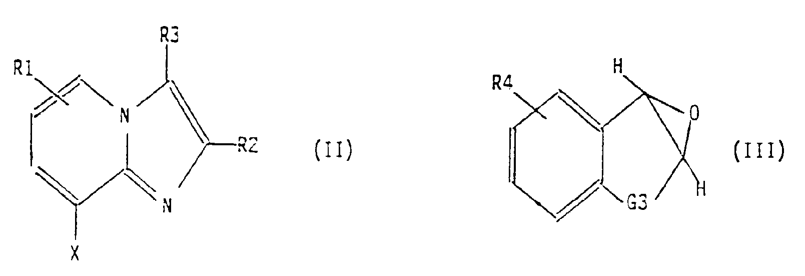 und gewünschtenfalls anschließend erhaltene Verbindungen der Formel I, worin R3 die Bedeutung Formyl bzw. Nitroso hat, zu den Verbindungen der Formel worin R3 die Bedeutung Hydroxymethyl bzw. Amino hat, reduziert, und/oder gewünschtenfalls anschließend erhaltene Salze in die freien Verbindungen oder erhaltene freie Verbindungen in die Salze überführt, wobei die Ausgangsverbindungen als solche oder in Form ihrer Salze eingesetzt werden und wobei R1, R2, R3, R4 und G3 die oben angegebenen Bedeutungen haben und X OH oder NH₂ bedeutet

2. Verfahren nach Anspruch 1 zur Herstellung der Verbindungen der Formel 1 nach Anspruch 1, worin
R1 Wasserstoff (H)
bedeutet,

3. Verfahren nach Anspruch 1 zur Herstellung der Verbindungen der Formel 1 nach Anspruch 1, worin
R1 Wasserstoff,
R2 Methyl,
R3 Wasserstoff, Methyl, Formyl, Hydroxymethyl, Cyanomethyl oder Amino und
R4 Wasserstoff, Chlor oder Fluor,
bedeutet.

4. Verfahren nach Anspruch 1 zur Herstellung der Verbindungen der Formel 1 nach Anspruch 1, worin
R1 Wasserstoff,
R2 Methyl,
R3 Wasserstoff, Methyl, Hydroxymethyl, Cyanomethyl oder Amino und
R4 Vasserstoff
bedeutet.

5. Verfahren nach Anspruch 1 zur Herstellung der Verbindungen der Formel I nach Anspruch 1, worin
R1 Wasserstoff, Chlor oder Fluor,
R2 Methyl oder Ethyl,
R3 Wasserstoff, Methyl, Formyl, Hydroxymethyl, Cyanomethyl oder Amino,
R4 Wasserstoff, Chlor oder Fluor
bedeutet.

6. Verfahren nach Anspruch 1 zur Herstellung der Verbindungen der Formel I nach Anspruch 1, worin
R1 Wasserstoff,
R2 Methyl,
R3 Wasserstoff, Methyl, Hydroxymethyl, Cyanomethyl oder Amino und
R4 Wasserstoff oder Fluor,
bedeutet.

## Claims (Claims for the following Contracting State(s) : AT, BE, CH, DE, FR, GB, IT, LI, LU, NL, SE)

1. Compounds of formula I 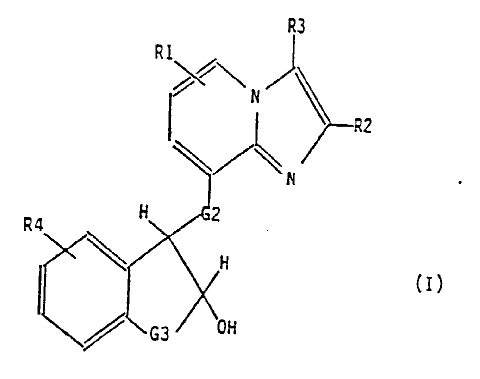 wherein
R1 denotes (H) or halogen,
R2 denotes 1-4C-alkyl,
R3 denotes hydrogen (H), 1-4C-alkyl, formyl, hydroxy-1-4C-alkyl, cyano-1 -4C-alkyl, nitroso or amino
R4 denotes hydrogen (H) or halogen,
G2 denotes O (oxygen) or NH,
G₃ denotes -CH₂- or -CH₂CH₂-,
G2 and OH being in trans-position to each other,
and their salts.

2. Compounds according to claim 1, wherein
R1 denotes hydrogen (H).

3. Compounds according to claim 1, wherein
R1 denotes hydrogen,
R2 denotes methyl,
R3 denotes hydrogen, methyl, formyl, hydroxymethyl, cyanomethyl or amino and
R4 denotes hydrogen, chlorine or fluorine.

4. Compounds according to claim 1, wherein
R1 denotes hydrogen,
R2 denotes methyl,
R3 denotes hydrogen, methyl, hydroxymethyl, cyanomethyl or amino and
R4 denotes hydrogen.

5. Compounds according to claim 1, wherein
R1 denotes hydrogen, chlorine or fluorine,
R2 denotes methyl or ethyl,
R3 denotes hydrogen, methyl, formyl, hydroxymethyl, cyanomethyl or amino and
R4 denotes hydrogen, chlorine or fluorine.

6. Compounds according to claim 1, wherein
R1 denotes hydrogen,
R2 denotes methyl,
R3 denotes hydrogen, methyl, hydroxymethyl, cyanomethyl or amino and
R4 denotes hydrogen or fluorine.

7. Process for the preparation of the compounds of formula I according to claim 1 and their salts, characterized in that imidazopyridines of formula II are reacted with bicycles of formula III 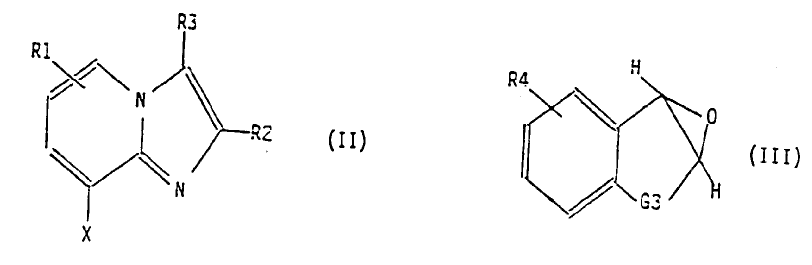 and, if desired, the compounds obtain of formula I, wherein R3 denotes formyl or nitroso are reduced to the compounds of formula I, wherein R3 denotes hydroxymethyl or amino and/or, if desired, the salts obtained are converted into the free compounds or the free compounds obtained are converted into the salts, with the starting compounds being used as such or in the form of their salts, wherein R1, R2, R3, R4 and G3 have the meanings given in claim 1 and X denotes OH or NH₂.

8. Drugs containing one or more compounds according to one or more of the claims 1 to 6 and/or their pharmacologically tolerated salts.

9. Compounds according to one or more of the claims 1 to 6 and their pharmacologically tolerated salts for use in the prophylaxis and treatment of gastrointestinal diseases.

10. The use of compounds according to one or more of the claims 1 to 6 and their pharmacologically tolerated salts for preparation of drugs for the prevention and treatment of gastrointestinal diseases.

## Claims (Claims for the following Contracting State(s) : ES, GR)

1. Process for the preparation of the compounds of formula I 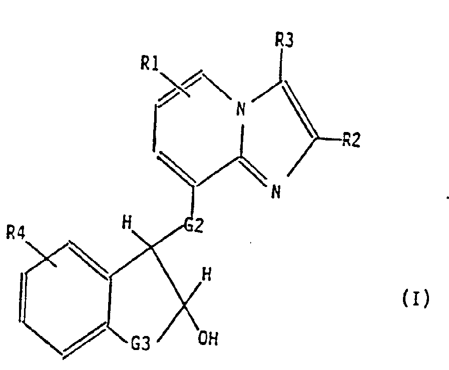 wherein
R1 denotes (H) or halogen,
R2 denotes 1-4C-alkyl,
R3 denotes hydrogen (H), 1-4C-alkyl, formyl, hydroxy-1-4C-alkyl, cyano-1 -4C-alkyl, nitroso or amino
R4 denotes hydrogen (H) or halogen,
G2 denotes O (oxygen) or NH,
G3 denotes -CH₂- or -CH₂CH₂₋,
G2 and OH being in trans-position to each other,
and their salts, characterized in that imidazopyridines of formula II are reacted with bicycles of formula III 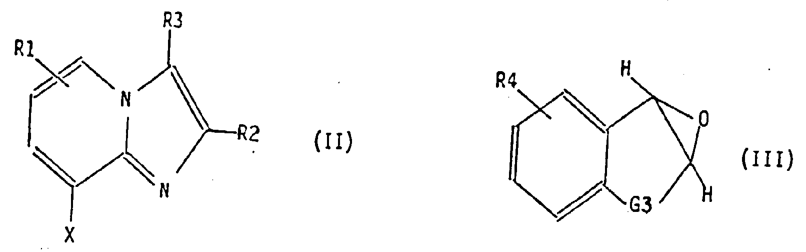 and, if desired, the compounds obtain of formula I, wherein R3 denotes formyl or nitroso are reduced to the compounds of formula I, wherein R3 denotes hydroxymethyl or amino and/or, if desired, the salts obtained are converted into the free compounds or the free compounds obtained are converted into the salts, with the starting compounds being used as such or in the form of their salts, wherein R1, R2, R3, R4 and G3 have the meanings given above and X denotes OH or NH₂.

2. Process according to claim 1 for the preparation of compounds of formula I according to claim 1, wherein R1 denotes hydrogen (H).

3. Process according to claim 1 for the preparation of compounds formula I according to claim 1, wherein
R1 denotes hydrogen,
R2 denotes methyl,
R3 denotes hydrogen, methyl, formyl, hydroxymethyl, cyanomethyl or amino and
R4 denotes hydrogen, chlorine or fluorine.

4. Process according to claim 1 for the preparation of compounds formula I according to claim 1, wherein
R1 denotes hydrogen,
R2 denotes methyl,
R3 denotes hydrogen, methyl, hydroxymethyl, cyanomethyl or amino and
R4 denotes hydrogen.

5. Process according to claim 1 for the preparation of compounds formula I according to claim I, wherein
R1 denotes hydrogen, chlorine or fluorine,
R2 denotes methyl or ethyl,
R3 denotes hydrogen, methyl, formyl, hydroxymethyl, cyanomethyl or amino and
R4 denotes hydrogen, chlorine or fluorine.

6. Process according to claim 1 for the preparation of compounds formula I according to claim 1, wherein
R1 denotes hydrogen,
R2 denotes methyl,
R3 denotes hydrogen, methyl, hydroxymethyl, cyanomethyl or amino and
R4 denotes hydrogen or fluorine.

## Revendications (Revendications pour l'(les) Etat(s) contractant(s) suivant(s) : AT, BE, CH, DE, FR, GB, IT, LI, LU, NL, SE)

1. Composés de formule 1: 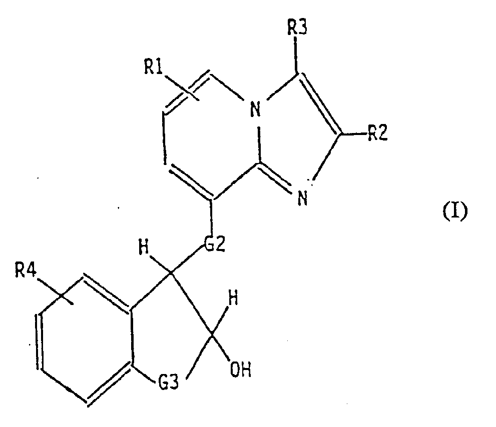 dans laquelle
R1 représente un atome d'hydrogène (H) ou d'halogène,
R2 représente un groupe alkyle en C₁₋₄
R3 représente un atome d'hydrogène (H) ou un groupe alkyle en C₁₋₄; formyle, hydroxy-alkyle en C₁,₄, cyano- alkyle en C₁₋₄, nitroso ou amino,
R4 représente un atome d'hydrogène (H) ou d'halogène,
G2 représente un atome d'oxygène (O) ou un groupe -NH-, et G3 représente un groupe -CH₂- ou -CH₂CH₂-,
G2 et le groupe -OH étant placés en trans l'un par rapport à l'autre, et sels de tels composés.

2. Composés conformes à la revendication 1, dans lesquels R1 représente un atome d'hydrogène (H).

3. Composés conformes à la revendication 1, dans lesquels
R1 représente un atome d'hydrogène,
R2 représente un groupe méthyle,
R3 représente un atome d'hydrogène ou un groupe méthyle, formyle, hydroxyméthyle, cyanométhyle ou ami-
no, et
R4 représente un atome d'hydrogène, de chlore ou de fluor.

4. Composés conformes à la revendication 1, dans lesquels
R1 représente un atome d'hydrogène,
R2 représente un groupe méthyle,
R3 représente un atome d'hydrogène ou un groupe méthyle, hydroxyméthyle, cyanométhyle ou amino, et
R4 représente un atome d'hydrogène.

5. Composés conformes à la revendication 1, dans lesquels
R1 représente un atome d'hydrogène, de chlore ou de fluor,
R2 représente un groupe méthyle ou éthyle,
R3 représente un atome d'hydrogène ou un groupe méthyle, formyle, hydroxyméthyle, cyanométhyle ou amino, et
R4 représente un atome d'hydrogène, de chlore ou de fluor.

6. Composés de formule 1, conformes à la revendication 1, dans lesquels
R1 représente un atome d'hydrogène,
R2 représente un groupe méthyle,
R3 représente un atome d'hydrogène ou un groupe méthyle, hydroxyméthyle, cyanométhyle ou amino, et R4 représente un atome d'hydrogène ou de fluor.

7. Procédé de préparation de composés de formule 1, conformes à la revendication 1, et de sels de tels composés, caractérisé en ce que l'on fait réagir une imidazopyridine de formule Il avec un composé bicyclique de formule III 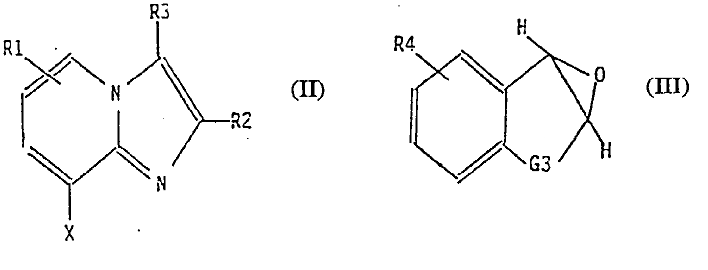 et l'on réduit ensuite, si on le souhaite, les composés obtenus de formule I où R3 représente un groupe formyle ou nitroso en les composés de formule I où R3 représente un groupe hydroxyméthyle ou amino, et/ou l'on transforme ultérieurement, si on le souhaite, les sels obtenus en composés libres ou les composés libres obtenus en sels, les composés de départ étant utilisés tels quels ou sous forme de sels, R1, R2, R3, R4 et G3 ayant les significations indiquées dans la revendication 1 et X représentant un groupe -OH ou -NH₂.

8. Médicament contenant un ou plusieurs composés, conformes à l'une ou plusieurs des revendications 1 à 6, et/ou un ou plusieurs sels admissibles en pharmacie de tels composés

9. Composés, conformes à l'une ou plusieurs des revendications 1 à 6, et sels admissibles en pharmacie de tels composés, destinés à être utilisés pour la prévention et le traitement de maladies gastrointestinales.

10. Emploi de composés, conformes à l'une ou plusieurs des revendications 1 à 6, et/ou de sels admissibles en pharmacie de tels composés, pour préparer des médicaments destinés à la prévention et au traitement de maladies gastro-intestinales.

## Revendications (Revendications pour l'(les) Etat(s) contractant(s) suivant(s) : ES, GR)

1. Procédé de préparation de composés de formule 1 : 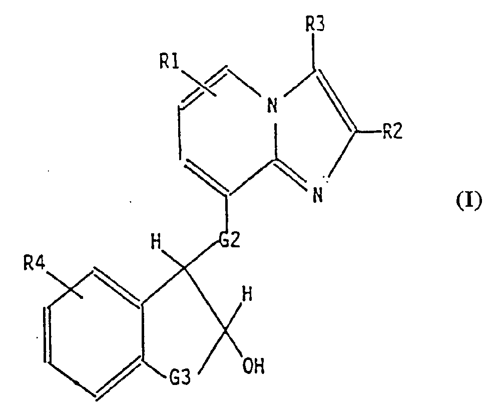 dans laquelle
R1 représente un atome d'hydrogène (H) ou d'halogène,
R2 représente un groupe alkyle en C₁₋₄,
R3 représente un atome d'hydrogène (H) ou un groupe alkyle en C₁₋₄, formyle, hydroxy-alkyle en C₁₋₄, cyano- alkyle en C₁₋₄, nitroso ou amino,
R4 représente un atome d'hydrogène (H) ou d'halogène,
G2 représente un atome d'oxygène (O) ou un groupe -NH-, et
G3 représente un groupe -CH₂- ou -CH₂CH₂-,
G2 et le groupe -OH étant placés en trans l'un par rapport à l'autre, et de sels de tels composés,
caractérisé en ce que l'on fait réagir une imidazopyridine de formule Il avec un composé bicyclique de formule III 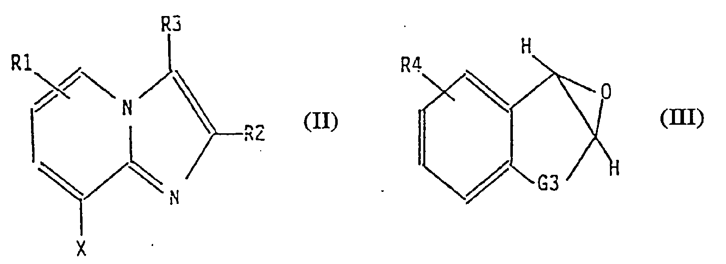 et l'on réduit ensuite, si on le souhaite, les composés obtenus de formule I où R3 représente un groupe formyle ou nitroso en les composés de formule I où R3 représente un groupe hydroxyméthyle ou amino, et/ou l'on transforme ultérieurement, si on le souhaite, les sels obtenus en composés libres ou les composés libres obtenus en sels, les composés de départ étant utilisés tels quels ou sous forme de sels, R1, R2, R3, R4 et G3 ayant les significations indiquées ci-dessus et X représentant un groupe -OH ou -NH₂.

2. Procédé, conforme à la revendication 1, de préparation de composés de formule 1, indiquée dans la revendication 1, où R1 représente un atome d'hydrogène (H).

3. Procédé, conforme à la revendication 1, de préparation de composés de formule 1, indiquée dans la revendication 1, où
R1 représente un atome d'hydrogène,
R2 représente un groupe méthyle,
R3 représente un atome d'hydrogène ou un groupe méthyle, formyle, hydroxyméthyle, cyanométhyle ou amino, et
R4 représente un atome d'hydrogène, de chlore ou de fluor.

4. Procédé, conforme à la revendication 1, de préparation de composés de formule 1, indiquée dans la revendication 1, où
R1 représente un atome d'hydrogène,
R2 représente un groupe méthyle,
R3 représente un atome d'hydrogène ou un groupe méthyle, hydroxyméthyle, cyanométhyle ou amino, et
R4 représente un atome d'hydrogène.

5. Procédé, conforme à la revendication 1, de préparation de composés de formule 1, indiquée dans la revendication 1, où
R1 représente un atome d'hydrogène, de chlore ou de fluor,
R2 représente un groupe méthyle ou éthyle,
R3 représente un atome d'hydrogène ou un groupe méthyle, formyle, hydroxyméthyle, cyanométhyle ou amino, et
R4 représente un atome d'hydrogène, de chlore ou de fluor.

6. Procédé, conforme à la revendication 1, de préparation de composés de formule 1, indiquée dans la revendication 1, où
R1 représente un atome d'hydrogène,
R2 représente un groupe méthyle,
R3 représente un atome d'hydrogène ou un groupe méthyle, hydroxyméthyle, cyanométhyle ou amino, et
R4 représente un atome d'hydrogène ou de fluor.
